# FASCICULE DE BREVET EUROPEEN

(11) **EP 0 523 183 B1**
(45) Date de publication et mention de la délivrance du brevet: **02.02.1994**
(21) Numéro de dépôt: 91908505.0
(22) Date de dépôt: 04.04.1991
(51) Int. Cl.: A61K 9/00, A61K 9/16, A61K 9/20, A61K 9/51

(54) **MICROSPHERES, LEUR PROCEDE DE PREPARATION ET LEUR UTILISATION**
MIKROKUGELN, VERFAHREN ZU IHRER HERSTELLUNG UND IHRE VERWENDUNG
MICROSPHERES, A PREPARATION METHOD THEREFOR AND USES THEREOF

(30) Priorité: 06.04.1990 FR 9004471
(43) Date de publication de la demande: 20.01.1993
(73) Titulaire: RHONE-POULENC RORER S.A., 92160 Antony (FR)
(72) Inventeur: SPENLEHAUER, Gilles, F-94230 Cachan (FR); VEILLARD, Michel, F-92330 Sceaux (FR); VERRECHIA, Thierry, F-94110 Arcueil (FR)
(74) Mandataire: Le Pennec, Magali
(86) Numéro de dépôt international: FR9100274
(87) Numéro de publication internationale: WO9115193

(56) Documents cités:
- EP-A- 0 134 318
- EP-A- 0 269 921
- GB-A- 2 077 693
- US-A- 4 330 338

## Description

La présente invention concerne de nouvelles formes pharmaceutiques. Elle concerne plus particulièrement des microsphères de principes actifs ayant un diamètre particulièrement faible de l'ordre d'environ un µm. Elle vise également un procédé de préparation desdites microsphères et leur utilisation.

Il est particulièrement important dans le domaine pharmaceutique de pouvoir disposer de formes pharmaceutiques se présentant sous des dimensions extrêmement réduites et ayant en plus une homogénéité de répartition remarquable. Ces exigences sont surtout importantes pour les formes pharmaceutiques destinées à une administration parentérale.

Certains principes actifs requièrent aussi un enrobage pour leur administration. Ainsi se pose un double problème obtenir des formes pharmaceutiques présentant un diamètre aussi faible que possible, le principe actif étant enrobé par un polymère, l'ensemble devant pouvoir être administré à l'homme ou à l'animal. Ce polymère doit aussi posséder des propriétés de biocompatibilité et de biodégradabilité remarquables.

Ce problème général est connu de l'industrie pharmaceutique depuis longtemps. Diverses descriptions de microparticules ont déjà été proposées comme par exemple dans le brevet US 4 330 338. Ce brevet qui ne répond pas au problème préalablement évoqué décrit un procédé de préparation de microsphères de polymère et leur addition subséquente à des principes actifs pharmaceutiques, lors de la préparation par exemple de comprimés.

Selon ce brevet, on prépare des microsphères de polymère, en réalisant une solution du polymère non hydrosoluble dans un solvant plus volatil que l'eau, puis on émulsionne cette solution dans une phase aqueuse éventuellement en présence d'un agent émulsifiant et enfin on évapore le solvant. Les microsphères obtenues présentent un diamètre réparti entre 0,1 et 20 µm. Elles sont utilisées pour enrober des principes actifs pharmaceutiques. Il est précisé dans ce brevet que pour obtenir le taux désiré d'enrobant, lorsque le polymère est insoluble dans l'eau, il est nécessaire d'ajouter à l'émulsion un agent tel que qu'un polymère hydrosoluble comme par exemple la méthyl cellulose ou la polyvinylpyrrolidone. Ces agents favorisant l'enrobage sont choisis parmi les composés solubles dans l'eau et acceptables pour l'ingestion. Malheureusement la plupart d'entre eux ne sont pas acceptables pour une administration par voie parentérale. Ce brevet ne décrit jamais l'enrobage d'un principe actif pharmaceutique sous la forme de microsphères et ne résoud donc pas le problème préalablement évoqué.

Il est également décrit dans le brevet EP 269 921 des microsphères de principes actifs enrobées d'un copolymère à base d'acide polylactique. Ces microsphères ont un diamètre moyen compris entre 0,1 et 10 µm. Elles sont obtenues par dissolution du polymère et du principe actif à enrober dans un solvant non miscible à l'eau, suivie d'une émulsion de la solution précédente dans une solution aqueuse contenant un émulsifiant. Cet émulsifiant est choisi parmi l'alcool polyvinylique, la polyvinylpyrrolidone, la carboxyméthylcellulose. Le seul émulsifiant exemplifié est l'alcool polyvinylique. Il est impossible pour l'administration à l'homme par voie injectable de maintenir des traces d'un tel composé.

Il est encore décrit selon le brevet GB 2 077 693 des microsphères présentant un diamètre inférieur à 150 µm et même compris entre 5 et 25 10⁻⁶ m qui sont obtenues par émulsion d'une solution organique du polymère biodégradable et biocompatible et d'une solution aqueuse d'un sel d'acide gras. Les sels d'acides gras ne sont pas biocompatibles et ce brevet exclut la présence seule d'émulsifiant tel que la gélatine.

La présente invention a permis d'obtenir des microsphères de principe actif enrobées par un polymère biodégradable et biocompatible et par une substance tensioactive elle aussi biodégradable et biocompatible.

Elle concerne les microsphères en tant que nouvelles compositions, leur procédé de préparation ainsi que leur utilisation.

Les microsphères selon l'invention sont constituées d'un principe actif pharmaceutique de base, d'un polymère biodégradable et d'une substance tensioactive. Le principe actif pharmaceutique est notamment choisi parmi :
- les agents antiinflammatoires (kétoprofène, ibuprofène, salicylés),
- les agents antibactériens (pénicillines. céphalosporines, les macrolides, les synergistines, les tétracyclines, les quinolones),
- les agents anticancéreux,
- les agents ayant une action sur le coeur (antiangoreux nitreux antiarythmiques, antihypertenseurs, bétabloquants, veinotoniques, vasodilatateurs),
- les agents de diagnostic.

Ces microsphères sont aussi constituées d'un polymère biodégradable et biocompatible choisi parmi :
- les homopolymères de l'acide lactique ou de l'acide glycolique ou les copolymères desdits acides,
- les polymères de l'acide polyhydroxybutyrique,
- les polylactones des acides gras contenant plus de douze atomes de carbone (polycaprolactones, polyvalérolactones),
- les polyorthoesters tels que décrit par HELLER, J.Polym. Sci., 18, 619, 1980
- les polyhydroxyesters d'acide gras ayant plus de douze atomes de carbone (polyhydroxyvalérate),
- les polyanhydrides.

On préfère parmi l'ensemble de ces composés utiliser les copolymères de l'acide lactique et de l'acide glycolique présentant un poids moléculaire compris entre 1000 et 200 000.

Ces microsphères sont aussi constituees d'un agent tensioactif biocompatible protéinique choisi parmi :
- la sérumalbumine,
- la fétuine,
- l'orosomucoïde,
- les glycoprotéines,
- les immunoglobulines,
- la gélatine,
- le collagène,

ou d'un phospholipide, d'un lipopolysacaccharide ou de sels biliaires tels que le cholate de sodium.

Ces microsphères présentent un diamètre particulaire compris entre 0,01 et 10 µm et de préférence entre 0,05 et 1 µm. Le principe actif peut indifféremment être situé dans le coeur de la microsphère mélangé avec le polymère biocompatible ou être situé à l'extérieur du coeur emprisonné dans le tensioactif. La situation du principe actif dépend fortement de son affinité pour le polymère ou pour le tensioactif.

Le procédé de préparation de ces microsphères consiste à mettre en solution le principe actif et le polymère dans un solvant organique, non miscible à l'eau, plus volatil que l'eau tel que par exemple les solvants halogénés et tout particulièrement le dichlorométhane, le chloroforme, le toluène, les alcools aliphatiques (éthanol, isopropanol), ou leurs mélanges.

On prépare à côté une solution aqueuse du tensioactif que l'on mélange à grande vitesse avec la solution précédente au moyen d'un homogénéisateur à haute pression (10⁵ à 11x10⁵ Pa). Cette technique permet d'éviter la présence de métaux lourds dans l'émulsion aqueuse de microsphères. La teneur en métaux lourds est avantageusement inférieure à 10 ppm.

On obtient alors une émulsion aqueuse contenant les microsphères qui subit ensuite une évaporation de façon à éliminer le solvant. Les microsphères obtenues en solution aqueuse peuvent être utilisées telles quelles ou peuvent subir une étape ultérieure de lyophilisation. Dans ce dernier cas on ajoute avantageusement un agent de lyophilisation tel que par exemple le mannitol ou le thréalose.

Selon une meilleure manière de mettre en oeuvre l'invention on utilise de préférence une quantité de polymère telle qu'elle représente une concentration pondérale par rapport au solvant comprise entre 0,01 et 20 % et encore plus préférentiellement comprise entre 1 et 10 %. On préfère aussi mettre en oeuvre au maximum 25 % de principe actif dans le milieu.

L'émulsion est réalisée en mettant en oeuvre de préférence :
- 1 à 50 % en poids de solvant contenant le polymère et le principe actif,
- 98,9 à 30 % en poids d'eau,
- 0,1 à 20 % en poids d'agent tensioactif.

Encore plus préférentiellement on met en oeuvre :
- 1 à 30 % en poids de solvant contenant le polymère et le principe actif,
- 98,9 à 50 % en poids d'eau,
- 0,1 à 20 % en poids d'agent tensioactif.

La solution aqueuse obtenue aprés évaporation du solvant contenant les microsphères est constituée de :
- 0,05 à 20 % en poids de principe actif,
- 0,1 à 40 % en poids de polymère,
- 0,2 à 20 % en poids d'agent tensioactif,
- 99,65 a 20 % en poids d'eau.

Elle est encore plus préférentiellement constituée de :
- 0,05 à 12 % en poids de principe actif,
- 0,1 à 25 % en poids de polymère,
- 0,1 à 20 % en poids d'agent tensioactif,
- 99,65 à 57 % en poids d'eau.

Cette solution est directement utilisable pour une utilisation parentérale.

La solution aqueuse obtenue peut aussi avantageusement subir une étape ultérieure de lyophilisation après addition d'environ 10 % en poids de mannitol par rapport au poids d'eau contenu dans la solution devant subir la lyophilisation.

L'invention sera plus complètement décrite à l'aide des exemples suivants qui ne doivent pas étre considérés comme limitatifs de l'invention.

### EXEMPLE 1

Une suspension à 1 % (P/P) de nanoparticules d'un copolymère des acides D,L lactique (37,5 % L et 37,5 % D) et glycolique (25%) (PLA37.5 GA25) et de spiramycine est préparée en dissolvant 0,5 g de ce polymère et 0,5 g de spiramycine dans 10 g de dichlorométhane. Cette solution est ensuite dispersée dans 50 g d'une solution aqueuse de cholate de sodium à 1 % (P/P). Une émulsion grossière est obtenue. Elle est recyclée pendant 3 minutes à l'aide d'un homogénéisateur haute pression type MICROFLUIDIX!. L'émulsion est alors débarrassée du dichlorométhane à l'aide d'un évaporateur rotatif à une pression de 50,5 cm de mercure à 20°C. Le pseudo-latex obtenu est constitué de nanoparticules d'un diamètre moyen de 60 +/-15 nm, et contient 12,6 % (P/P) de spiramycine.

### EXEMPLE 2

Une suspension à 7 % (P/P) de nanoparticules de PLA37. GA25 est préparée en dissolvant 3,5 g du mélange de l'exemple 1 dans 45 g de dichlorométhane et en suivant le protocole décrit dans l'exemple 1. Les particules ont un diamètre moyen de 270 +/- 50 nm.

### EXEMPLE 3

Une suspension à 15 % (P/P) de nanoparticules de poly-(L)lactique est obtenue en dissolvant 7,5 g du mélange de l'exemple 1 dans du dichlorométhane et suivant le protocole décrit dans l'exemple 1.

### EXEMPLE 4

Une suspension à 1 % (P/P) de nanoparticules d'acide polyhydroxybutyrique et de phénoxyméthylpénicilline est obtenue suivant le protocole décrit dans l'exemple 1 en remplaçant le cholate de sodium par de la sérum albumine, et la spiramycine par 0,21 g de pénicilline V acide (phénoxyméthylpénicilline). Les nanoparticules contiennent 7,2 % (P/P) d'antibiotique.

### EXEMPLE 5

Une suspension à 1 % (P/P) de nanoparticules de spiramycine et de polyanhydride est obtenue en suivant le protocole décrit dans l'exemple 1 en remplaçant le cholate de sodium par un mélange gélatine/Pluronic F68 (50/50 P/P).

### EXEMPLE 6

Une suspension à 1 % (P/P) de nanoparticules de spiramycine et de PLA37.5 GA25 est obtenue en suivant le protocole décrit dans l'exemple 1 en remplaçant le cholate de sodium par de la lécithine purifiée. Une observation en microscopie électronique à transmission révèle la présence de feuillets de phospholipides entourant les nanoparticules de polymère.

### EXEMPLE 7

Une suspension à 1 % (P/P) de nanoparticules de spiramycine et de poly-ε-caprolactone est obtenue en suivant le protocole décrit dans l'exemple 1 en remplaçant le cholate de sodium par du collagène.

### EXEMPLE 8

Une suspension à 1 % (P/P) de nanoparticules de spiramycine et de PLA37.5 GA25 est obtenue en suivant le protocole décrit dans l'exemple 1 en remplaçant le cholate de sodium par de la fétuine.

### EXEMPLE 9

Une suspension à 1 % (P/P) de nanoparticules de spiramycine et d'un copolymère des acides hydroxybutyrique et valérique est obtenue en suivant le protocole décrit dans l'exemple 1 en remplaçant le cholate de sodium par de l'orosomucoïde.

### EXEMPLE 10

Une suspension à 1 % (P/P) de nanoparticules de spiramycine et de PLA37.5 GA25 contenant de l'huile de coton (Mygliol 812) est obtenue suivant le protocole décrit dans l'exemple 1 en additionnant 0,1 g d'huile dans la solution de polymère dans le dichlorométhane.

### EXEMPLE 11

Une suspension à 1 % (P/P) de nanoparticules de spiramycine et de PLA37.5 GA25 est obtenue en suivant le protocole décrit dans l'exemple 1 en remplaçant le cholate de sodium par une immunoglobuline.

### EXEMPLE 12

Une suspension à 1 % (P/P) de nanoparticules de spiramycine et de PLA37.5 GA25 est obtenue en suivant le protocole décrit dans l'exemple 1 en remplaçant le cholate de sodium par un lipopolysaccharide de paroi bactérienne.

### EXEMPLE 13

Une suspension à 0,1 % (P/P) de nanoparticules de spiramycine et de PLA37.5 GA25 est obtenue en suivant le protocole décrit dans l'exemple 1 en remplaçant le cholate de sodium par un mélange de lécithine/ ganglioside M1 (5/1, mol/mol).

### EXEMPLE 14

Une suspension à 0,1 % (P/P) de nanoparticules de spiramycine et de PLA37.5 GA25 est obtenue en suivant le protocole décrit dans l'exemple 1 en remplaçant le cholate de sodium par une lipoproteïne haute densité.

### EXEMPLE 15

Une suspension à 1 % (P/P) de nanoparticules de spiramycine et de Poly-(L) lactique est obtenue en suivant le protocole de l'exemple 1 mais en utilisant une solution aqueuse de cholate de sodium à 0,05 %, les nanoparticules ont un diamètre moyen de 280 +/- 60 nm.

### EXEMPLE 16

Une suspension à 1 % (P/P) de nanoparticules de spiramycine et de Poly-(D,L) lactique est obtenue en suivant le protocole de l'exemple 1 mais en utilisant une solution aqueuse de sérum albumine à 0,05 %, les nanoparticules ont un diamètre moyen de 320 +/- 60 nm.

### EXEMPLE 17

Une suspension à 1 % (P/P) de nanoparticules de spiramycine et de Poly-(D,L) lactique est obtenue en suivant le protocole de l'exemple 1 mais en utilisant une solution aqueuse de sérum albumine à 3 %, les nanoparticules ont un diamètre moyen de 80 +/- 20 nm.

## Revendications

1. Microsphères biocompatibles caractérisées en ce qu'elles sont constituées d'un ou plusieurs principes actifs, d'un polymère biodégradable et biocompatible et d'une substance tensioactive elle aussi biodégradable et biocompatible, contenant moins de 10 ppm de métaux lourds.

2. Microsphères biocompatibles selon la revendication 1 caractérisées en ce qu'elles présentent un diamètre particulaire compris entre 0,05 et 1 µm.

3. Microsphères selon la revendication 1 caractérisées en ce que le polymère biodégradable et biocompatible est choisi parmi :
- les homopolymères de l'acide lactique ou de l'acide glycolique ou les copolymères desdits acides,
- les polymères de l'acide polyhydroxybutyrique,
- les polylactones des acides gras contenant plus de douze atomes de carbone (polycaprolactones, polyvalérolactones),
- les polyorthoesters,
- les polyhydroxyesters d'acide gras ayant plus de douze atomes de carbone (polyhydroxyvalérate),
- les polyanhydrides.

4. Microsphères selon la revendication 1 caractérisées en ce que l'agent tensioactif biocompatible est choisi parmi les composés protéiniques suivants :
- la sérumalbumine,
- la fétuine,
- l'orosomucoïde,
- les glycoprotéines,
- les immunoglobulines,
- la gélatine,
- le collagène,
ou les phospholipides et les lipopolysaccharides.

5. Procédé de préparation des microsphères selon l'une quelconque des revendications précédentes caractérisé en ce qu'on prépare une solution du polymère et du principe actif dans un solvant non miscible à l'eau, plus volatil que l'eau que l'on mélange au moyen d'un homogénéisateur à haute pression avec une solution aqueuse du tensioactif, suivi d'une évaporation du solvant.

6. Procédé de préparation selon la revendication 5 caractérisé en ce que le solvant est choisi parmi les solvants aliphatiques halogénés, les alcools et les solvants aromatiques.

7. Procédé selon la revendication 5 caractérisé en ce que le mélange est effectué au moyen d'un homogénéisateur à pression comprise entre 10⁵ et 11x10⁵ Pa.

8. procédé selon la revendication 5 caractérisé en ce qu'on utilise une concentration pondérale en polymère par rapport au solvant comprise entre 0,01 et 20 % en poids, et de préférence entre 1 et 10 %.

9. Procédé selon la revendication 5 caractérisé en ce que le mélange est composée de :
- 1 à 50 % en poids de solvant contenant le polymère et le principe actif,
- 98,9 à 30 % en poids d'eau,
- 0,1 à 20 % d'agent tensioactif.

10. Procédé selon la revendication 9 caractérisé en ce que le mélange est composée de :
- 1 à 30 % en poids de solvant contenant le polymère et le principe actif,
- 98,9 à 50 % en poids d'eau,
- 0,1 à 20 % en poids d'agent tensioactif.

11. Compositions injectables contenant un ou plusieurs principes actifs sous forme de microsphères selon l'une quelconque des revendications 1 à 4 caractérisées en ce qu'elles sont constituées de :
- 0,05 à 20 % en poids de principe actif,
- 0,1 à 40 % en poids de polymère,
- 0,2 à 20 % en poids d'agent tensioactif,
- 99,65 à 20 % en poids d'eau.

12. Compositions injectables contenant un ou plusieurs principes actifs sous forme de microsphères selon l'une quelconque des revendications 1 à 4 caractérisées en ce qu'elles contiennent :
- 0,05 à 12 % en poids de principe actif,
- 0,1 à 25 % en poids de polymère,
- 0,1 à 20 % en poids d'agent tensioactif,
- 99,65 à 57 % en poids d'eau.

## Patentansprüche

1. Biokompatible Mikrokugeln, dadurch gekennzeichnet, daß sie aus einem oder mehreren Wirkstoffen, einem biodegradablen und biokompatiblen Polymer und einem oberflächenaktiven Stoff bestehen, der ebenfalls biodegradabel und biokompatibel ist und weniger als 10 ppm Schwermetalle enthält.

2. Biokompatible Mikrokugeln nach Anspruch 1, dadurch gekennzeichnet, daß sie einen Durchmesser der Teilchen zwischen 0,05 und 1 µm aufweisen.

3. Mikrokugeln nach Anspruch 1, dadurch gekennzeichnet, daß das biodegradable und biokompatible Polymer ausgewählt wird unter:
- den Homopolymeren von Milchsäure oder Glykolsäure oder den Copolymeren der genannten Säuren,
- den Polymeren von Polyhydroxybuttersäure,
- den Polylactonen der Fettsäuren, die mehr als 12 Kohlenstoffatome enthalten (Polycaprolactone. Polyvalerolactone).
- den Polyorthoestern.
- den Polyhydroxyestern von Fettsäuren mit mehr als 12 Kohlenstoffatomen (Polyhydroxyvalerate),
- den Polyanhydriden.

4. Mikrokugeln nach Anspruch 1. dadurch gekennzeichnet, daß das biokompatible oberflächenaktive Mittel unter den folgenden Proteinverbindungen ausgewählt wird:
- Serumalbumin,
- Fetuin,
- Orosomucoid,
- den Glycoproteinen,
- den Immunoglobulinen,
- Gelatine,
- Collagen,
oder den Phospholipiden und den Lipopolysacchariden.

5. Verfahren zur Herstellung der Mikrokugeln nach Irgendeinem der vorstehenden Ansprüche, dadurch gekennzeichnet, daß man eine Lösung des Polymers und des Wirkstoffes in einem mit Wasser nicht mischbaren Lösungsmittel herstellt, das flüchtiger ist als Wasser, daß man sie mit Hilfe eines Homogenisators unter hohem Druck mit einer wäßrigen Lösung des oberflächenaktiven Mittels vermischt und man danach das Lösungsmittel verdampft.

6. Verfahren zur Herstellung nach Anspruch 5. dadurch gekennzeichnet, daß das Lösungsmittel unter den halogenierten aliphatischen Lösungsmitteln, den Alkoholen und den aromatischen Lösungsmitteln ausgewählt wird.

7. Verfahren nach Anspruch 5. dadurch gekennzeichnet, daß das Mischen mit Hilfe eines Homogenisators unter einem Druck zwischen 10⁵ und 11·10⁵ Pa durchgeführt wird.

8. Verfahren nach Anspruch 5, dadurch gekennzeichnet, daß man eine Gewichts-Konzentration an Polymer in bezug auf das Lösungsmittel zwischen 0,01 und 20 Gew.-%. und vorzugsweise zwischen 1 und 10 Gew.-% verwendet.

9. Verfahren nach Anspruch 5, dadurch gekennzeichnet, daß die Mischung zusammengesetzt ist aus:
- 1 bis 50 Gew.-% Lösungsmittel, enthaltend das Polymer und den Wirkstoff,
- 98,9 bis 30 Gew.-% Wasser,
- 0,1 bis 20 Gew.-% oberflächenaktives Mittel.

10. Verfahren nach Anspruch 9, dadurch gekennzeichnet, daß die Mischung zusammengesetzt ist aus:
- 1 bis 30 Gew.-% Lösungsmittel, enthaltend das Polymer und den Wirkstoff,
- 98,9 bis 50 Gew.-% Wasser,
- 0,1 bis 20 Gew.-% oberflächenaktives Mittel.

11. Injizierbare Zusammensetzungen. enthaltend einen oder mehrere Wirkstoffe in Form von Mikrokugeln nach einem der Ansprüche 1 bis 4. dadurch gekennzeichnet, daß sie bestehen aus:
- 0,05 bis 20 Gew.-% Wirkstoff,
- 0,1 bis 40 Gew.-% Polymer,
- 0.2 bis 20 Gew.-% oberflächenaktivem Mittel,
- 99,65 bis 20 Gew.-% Wasser.

12. Injizierbare Zusammensetzungen, enthaltend einen oder mehrere Wirkstoffe in Form von Mikrokugeln nach einem der Ansprüche 1 bis 4, dadurch gekennzeichnet, daß sie enthalten:
- 0,05 bis 12 Gew.-% Wirkstoff,
- 0,1 bis 25 Gew.-% Polymer,
- 0,1 bis 20 Gew.-% oberflächenaktivem Mittel.
- 99,65 bis 57 Gew.-% Wasser.

## Claims

1. Biocompatible microspheres characterized in that they consist of one or more active ingredients, a biodegradable and biocompatible polymer and a surface-active substance which is also biodegradable and biocompatible, containing less than 10 ppm of heavy metals.

2. Biocompatible microspheres according to claim 1, characterized in that they have a particle diameter of between 0.05 and 1 µm.

3. Microspheres according to claim 1, characterized in that the biodegradable and biocompatible polymer is chosen from:
- homopolymers of lactic acid or glycolic acid or copolymers of the said acids,
- polymers of polyhydroxybutyric acid,
- polylactones of fatty acids containing more than twelve carbon atoms (polycaprolactones, polyvalerolactones),
- polyorthoesters,
- polyhydroxyesters of fatty acid having more than twelve carbon atoms (polyhydroxyvalerate),
- polyanhydrides.

4. Microspheres according to claim 1, characterized in that the biocompatible surface-active agent is chosen from the following protein compounds:
- serum albumin,
- fetuin,
- orosomucoid,
- glycoproteins,
- immunoglobulins, gelatin,
- collagen,
or phospholipids and lipopolysaccharides.

5. Process for preparing the microspheres according to any one of the preceding claims, characterized in that a solution of the polymer and the active ingredient is prepared in a water-immiscible solvent, more volatile than water, which is mixed by means of a high-pressure homogenizer with an aqueous solution of the surface-active agent, followed by evaporation of the solvent.

6. Preparation process according to claim 5, characterized in that the solvent is chosen from halogenated aliphatic solvents, alcohols and aromatic solvents.

7. Process according to claim 5, characterized in that the mixing is performed by means of a homogenizer at a pressure of between 10⁵ and 11x10⁵ Pa.

8. Process according to claim 5, characterized in that there is used a polymer concentration by weight relative to the solvent of between 0.01 and 20 % by weight, and preferably between 1 and 10 %.

9. Process according to claim 5, characterized in that the mixture is composed of:
- 1 to 50 % by weight of solvent containing the polymer and the active ingredient,
- 98.9 to 30 % by weight of water,
- 0.1 to 20 % of surface-active agent.

10. Process according to claim 9, characterized in that the mixture is composed of:
- 1 to 30 % by weight of solvent containing the polymer and the active ingredient,
- 98.9 to 50 % by weight of water,
- 0.1 to 20 % by weight of surface-active agent.

11. Injectable compositions containing one or more active ingredients in the form of microspheres according to any one of claims 1 to 4, characterized in that they consist of:
- 0.05 to 20 % by weight of active ingredient,
- 0.1 to 40 % by weight of polymer,
- 0.2 to 20 % by weight of surface-active agent,
- 99.65 to 20 % by weight of water.

12. Injectable compositions containing one or more active ingredients in the form of microspheres according to any one of claims 1 to 4, characterized in that they contain:
- 0.05 to 12 % by weight of active ingredient,
- 0.1 to 25 % by weight of polymer,
- 0.1 to 20 % by weight of surface-active agent,
- 99.65 to 57 % by weight of water.
